# EUROPEAN PATENT APPLICATION

(11) **EP 2 204 454 A1**
(43) Date of publication of application: **07.07.2010**
(21) Application number: 10155902.9
(22) Date of filing: 04.05.2006
(51) Int. Cl.: C12Q 1/00, C12Q 1/70, C12Q 1/68, A61K 49/00, C07H 21/04, C07K 16/00, G01N 33/50

(54) **Predicting treatment response in cancer subjects**

(30) Priority: 04.05.2005 US 594763 P
(62) Divisional of application: 06759098.4
(71) Applicant: University of South Florida, Tampa, FL 33620-7900 (US)
(72) Inventor: Bepler, Gerold, Tierra Verde, FL 33715 (US)
(74) Representative: Fleck, Barbara

(57) **Abstract**

The invention relates to methods of determining an appropriate cancer therapy for a subject based on intratumoral expression levels of a gene , such as the RRM1 or ERCC1 gene. Compositions and kits useful in the methods are also provided. Moreover, methods employing cell lines with differential expression of RRM1 and ERCC1 are used in screening compounds.

SEQUENCE LISTING

<110> University of South Florida

<120> Predicting treatment response in cancer subjects

<130> PC780916EPA

<141> 2007-11-06

<150> PCT/US2006/017277
<151> 2006-05-04

<160> 3

<170> PatentIn version 3.3

<210> 1
<211> 64
<212> DNA
<213> artifical sequence

<220>
<221> misc_feature
<223> Designed oligonucleotide containing two 19 nucleotide RRM1 target
sequences for use in the generation of the pSUPER-siRRM1
construct.

<400> 1
gatccccgac gctagagcgg tcttatttca agagaataag accgctctag cgtctttttg 60

gaaa 64

<210> 2
<211> 64
<212> DNA
<213> artifical sequence

<220>
<221> misc_feature
<223> Designed oligonucleotide containing two 19 nucleotide RRM1 target
sequences for use in the generation of the pSUPER-siRRM1
construct.

<400> 2
agcttttcca aaaagacgct agagcggtct tattctcttg aaataagacc gctctagcgt 60

cggg 64

<210> 3
<211> 19
<212> DNA
<213> artifical sequence

<220>
<221>  misc_feature
<223>  Artifical sequence with no similarity to any known gene. Used in
      the production of the control H23siCt cell line.

<400>  3
gctaatagcg cggagtctt                                    19

## Description

### RELATED APPLICATIONS

This application claims the benefit of U.S. Provisional Application No. 60/594,763, filed May 4, 2005, which is incorporated herein by reference in its entirety.

### GOVERNMENT SUPPORT

This invention was made with government support under R01-CA102726 and R21-CA106616 awarded by the National Cancer Institute. The government has certain rights in the invention.

### TECHNICAL FIELD

The invention relates to methods of determining an appropriate cancer therapy for a subject based on intratumoral expression levels of a gene, such as the RRMI or ERCC1 gene.

### BACKGROUND

Two molecules involved in DNA synthesis and damage repair, the ribonucleotide reductase subunit M1 (RRM1) gene and the excision repair cross-complementing 1 (ERCC1) gene, are important in the pathogenesis of cancer. RRM1 and ERCC1 are targets of common chemotherapeutic agents. RRM1 is the molecular target of gemcitabine, and ERCC1 is crucial for platinum-induced DNA adduct repair. Gemcitabine and platinum-containing agents, such as cisplatin and carboplatin, are among the most frequently used in cancer therapy.

Historically, chemotherapy has had limited benefit to cancer patients. Typically, only about 25% of patients with epithelial malignancies, such as lung cancer, breast cancer, colorectal cancer, head and neck cancer, and ovarian cancer benefit from the therapy. However, after failure of initial therapy, a second round of chemotherapy is often effective, which suggests that sensitivity or resistance to such treatment is a function of specific characteristics of the tumor that are determined by molecules involved in the metabolic pathways of chemotherapeutic agents. Thus, it is desirable to develop methods that can be used to predict or assess the sensitivity of tumors to chemotherapeutic agents.

### SUMMARY

It has been discovered that expression levels of RRM1 or ERCC1 can be predictive of a patient's response to a cancer therapy. This discovery has led to the development of a test that is predictive of tumor response to therapy. Therapies selected based on the test results have proven to yield substantially better outcome for cancer patients than the currently used random selection of treatments. The test requires that a tumor specimen be collected and processed to determine the intratumoral levels of RRM1 or ERCC or both. Low levels of expression of RRM1 were found to correlate with better treatment response with antimetabolites, and low levels of expression of ERCC1 were found to correlate with better treatment response with platinum-containing agents. Patient with tumors exhibiting RRM1 expression in the highest or lowest quartile of a reference population were found to respond best to chemotherapies including an antitubulin agent.

In one aspect, the invention features a method for assessing a subject for an appropriate chemotherapy. The method includes providing a tumor sample from the subject, determining the RRM1 expression level in the tumor sample, and determining an appropriate chemotherapy based on the RRM1. expression level. If the RRM1 expression level is less than or equal to the median RRM1 expression level of a reference cohort, then a chemotherapy including an antimetabolite is determined to be appropriate, and if the RRM1 expression level is greater than the median RRM1 expression level of the reference cohort, then a chemotherapy lacking an antimetabolite is determined to be appropriate. The method can further include administering the appropriate chemotherapy to the subject. For example, if the RRM1 expression level is determined to be less than or equal to the median RRM1 expression level of the reference cohort, the subject can be administered an antimetabolite, such as gemcitabine or pemetrexed.

In some embodiments, the method includes administering a second chemotherapeutic agent to the subject, such as an antitubulin or platinum-containing agent. The antitubulin can be, for example, a taxane, such as paclitaxel or docetaxel, or a vinca alkaloid, such as vinorelbine, vincristine, vinblastine, vinflunine, or vindesine. The platinum-containing agent can be, for example, carboplatin, cisplatin, or oxaliplatin. In another embodiment, the method includes administering radiation therapy to the subject in addition to the appropriate chemotherapeutic agent.

In some embodiments, the subject who is assessed by the method described above has an epithelial malignancy, such as a lung cancer (*e*.*g*., a non-small-cell lung cancer), breast cancer, colorectal cancer, head and neck cancer, or ovarian cancer.

The method described above can also include determining the ERCC1 expression level in the tumor sample and further determining an appropriate chemotherapy based on both the RRM1 and ERCC1 expression levels. If the ERCC1 expression level is less than or equal to the median ERCC1 expression level of the reference cohort, then a chemotherapy comprising a platinum-containing agent is determined to be appropriate, and if the ERCC1 expression level is greater than the median ERCC1 expression level of the reference cohort., then a chemotherapy lacking a platinum-containing agent is determined to be appropriate. For example, the platinum-containing agent can be cisplatin, carboplatin, or oxaliplatin. The method can further include administering the appropriate chemotherapeutic agent to the subject.

The RRM1 and ERCC1 expression levels can be determined by RT-PCR, Northern blot, Western blot, or immunohistochemistry assay.

In another aspect, the invention features a method for assessing a subject for an appropriate chemotherapy by providing a tumor sample from the subject, determining the ERCC expression level in the tumor sample, and determining an appropriate chemotherapy based on the ERCC1 expression level. If the ERCC1 expression level is determined to be less than or equal to the median ERCC1 expression level of a reference cohort, then a chemotherapy comprising a platinum-containing agent is determined to be appropriate. If the ERCC1 expression level is greater than the median ERCC1 expression level of a reference cohort, then a chemotherapy lacking a platinum-containing agent is determined to be appropriate.

In some embodiments, the method further includes determining the RRM1. expression level in the tumor sample and determining an appropriate chemotherapy based on both the ERCC1 and RRM1 expression levels. If the RRM1 expression level is less than or equal to the median RRM1 expression level of the reference cohort, then a chemotherapy comprising an antimetabolite is determined to be appropriate, and if the RRM1 expression level is greater than the median RRM1 expression level of the reference cohort, then a chemotherapy lacking an antimetabolite is determined to be appropriate.

Also described herein is an alternative method for assessing a subject for an appropriate chemotherapy by providing a tumor sample from the subject, determining the RRM1 expression level in the tumor sample, and determining an appropriate chemotherapy based on the RRM1 expression level. By this method, if the RRM1 expression level is less than or equal to the RRM1 expression level of the lowest quartile of a reference cohort, or greater than or equal to the RRM1 expression level of the highest quartile of a reference cohort, then a chemotherapy comprising an antitubulin is determined to be appropriate. If the RRM1 expression level is determined not to be in the lowest or highest quartile of the reference cohort, then a chemotherapy lacking an antitubulin is determined to be appropriate. The antitubulin can be, for example, a taxane, such as paclitaxel or docetaxel, or a vinca alkaloid, such as vinorelbine, vincristine, vinblastine, vinflunine, or vindesine.

In some embodiments, the method further includes determining the ERCC1 expression level in the tumor sample and determining the chemotherapy based on both the RRM1 and ERCC1 expression levels. If the ERCC1 expression level is less than or equal to the median ERCC1 expression level of the reference cohort, then a chemotherapy comprising a platinum-containing agent is appropriate, and if the ERCC1 expression level is greater than the median ERCC1 expression level of the reference cohort, then a chemotherapy lacking a platinum-containing agent is appropriate. The platinum-containing agent can be, for example, cisplatin, carboplatin, or oxaliplatin.

In some embodiments, the method further includes administering the appropriate chemotherapy to the subject. In another embodiment, the method includes administering radiation therapy to the subject.

In one aspect, the invention features a method for assessing the efficacy of a composition containing a chemotherapeutic agent based on RRM1 expression levels. The method includes (i) providing a first cell line and a second cell line, wherein the first cell line expresses higher levels of RRM1 than the second cell line; (ii) contacting the first and second cell lines with the composition for a time sufficient to affect cell viability; and (iii) assaying the first and second cell lines for an effect on cell viability. A decrease in cell viability in the second cell line as compared to the first cell line indicates that the composition is likely to be efficacious against a tumor expressing lower levels of RRM1. A decrease in cell viability in the first cell line as compared to the second cell line indicates that the composition is likely to be efficacious against a tumor expressing higher levels of RRM1. In some embodiments, the composition includes one or more of an antimetabolite (*e*.*g*., gemcitabine or pemetrexed), an antitubulin, or a platinum-containing agent.

In another aspect, the invention features a method for assessing the efficacy of a composition containing a chemotherapeutic agent based on ERCC1 expression levels. The method includes (i) providing a first cell line and a second cell line, wherein the first cell line expresses higher levels of ERCC1 than the second cell line; (ii) contacting the first and second cell lines with the composition for a time sufficient to affect cell viability; and (iii) assaying the first and second cell lines for an effect on cell viability. A decrease in cell viability in the second cell line as compared to the first cell line indicates that the composition is likely to be efficacious against a tumor expressing lower levels of ERCC1. A decrease in cell viability in the first cell line as compared to the second cell line indicates that the composition is likely to be efficacious against a tumor expressing higher levels of ERCC1. In some embodiments, the composition includes one or more of an antimetabolite (*e*.*g*., gemcitabine or pemetrexed), an antitubulin, or a platinum-containing agent.

In yet another aspect, the invention features a kit that includes a reagent for assaying RRM1 expression in a tissue sample from a patient, and an instruction sheet. In some embodiments, the reagent for assaying RRM1 expression includes a premeasured portion of a reagent selected from the group selected from an oligo-dT primer, a forward primer that hybridizes to an RFM1 cDNA, a reverse primer that hybridizes to an RRM1 cDNA, a reverse transcriptase, a DNA polymerase, buffers, and nucleotides. In another embodiment, the reagent for assaying RRM1 expression includes a premeasured portion of an anti-RRM1 antibody and buffers for performing a Western blot or immunohistochemistry assay. The kit can also include a reagent for processing a tissue sample, such as a biopsy tissue sample, from a patient. In some embodiments, the kit also includes a reagent for assaying ERCC1 expression in the tissue sample. The reagent for assaying ERCC1 expression can be a premeasured portion of forward and reverse primers that hybridize to an ERCC1 cDNA, or an anti-ERCC1 antibody.

In one aspect, the invention features a kit that includes a reagent for assaying ERCC1 expression in a tissue sample from a patient, and an instruction sheet. The reagent for assaying ERCC1 expression can be a premeasured portion of a reagent selected from the group selected from an oligo-dT primer, a forward primer that hybridizes to an ERCC1 cDNA, a reverse primer that hybridizes to an ERCC1 cDNA, a reverse transcriptase, a DNApolymerase, buffers, and nucleotides, In another embodiment, the reagent for assaying ERCC1 expression includes a premeasured portion of an anti-ERCC1 antibody and buffers for performing a Western blot or immunohistochemistry assay. The kit can also include a reagent for processing a tissue sample, such as a biopsy tissue sample, from a patient. In some embodiments, the kit also includes a reagent for assaying RRM1 expression in the tissue sample. The reagent for assaying RRM1 expression can be a premeasured portion of forward and reverse primers that hybridize to an RRM1 cDNA, or an anti-RRM1 antibody.

In another aspect, the invention features a kit that includes a reagent for assaying RRM1 expression in a tissue sample from a patient, a reagent for assaying ERCC1 expression in a tissue sample from a patient, and an instruction sheet. In some embodiments, the reagents for assaying RRM1 and ERCC1 expression levels include a premeasured portion of a reagent selected from the group selected from an oligo-dT primer, forward and reverse primers that hybridizes to an RRM1 cDNA or an ERCC1 eDNA, a reverse transcriptase, a DNApolyznerase, buffers, and nucleotides. In another embodiment, the reagents for assaying RRM1 and ERCC1 expression include a premeasured portion of an anti-RRM1 antibody and an anti-ERCC1 antibody and buffers for performing Western blot or immunohistochemishy assays. The kit can also include a reagent for processing a tissue sample, such as a biopsy tissue sample, from a patient.

The details of one or more embodiments of the invention are set forth in the accompanying drawings and the description below. Other features, objects, and advantages of the invention will be apparent from the description and drawings, and from the claims.

### DESCRIPTION OF DRAWINGS

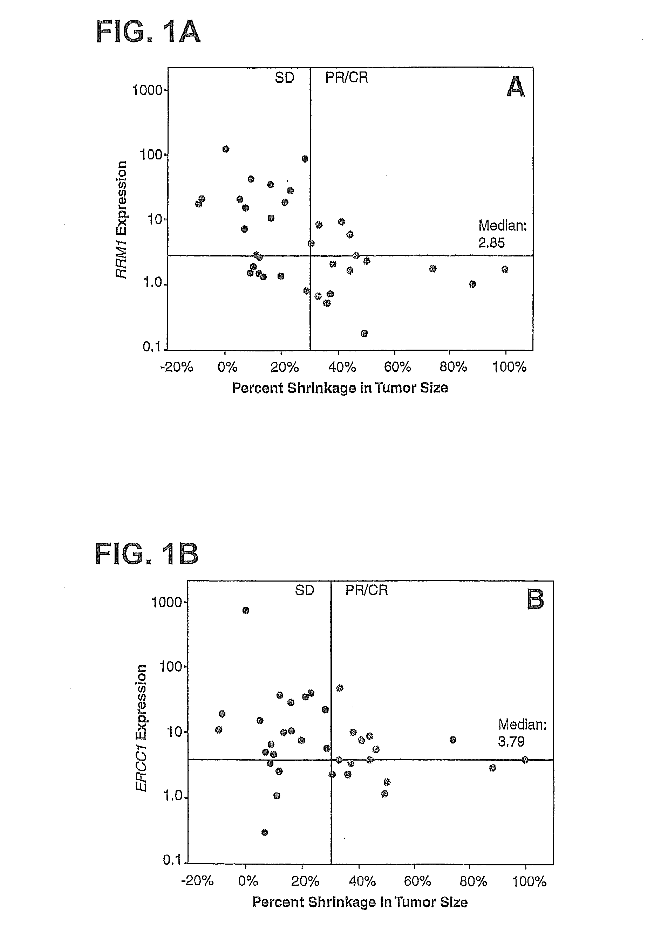

FIGs. 1A and 1B are a scatter plot of RRM1 (FIG. 1A) and ERCC1 (FIG. 1B) expression in relation to the percent change in tumor size after two cycles of gemcitabine and carboplatin chemotherapy in 35 patients with locally advanced non-small-cell lung carcinoma (NSCLC), Black dots indicate patients with less than 30% tumor shrinkage (SD), and gray dots indicate patients with greater than 30% tumor shrinkage (PR/CR). The Spearman correlation coefficient was r = -0.498 (p = 0.002) for RRM1 and r = -0.293 (p = 0.099) for ERCC1.

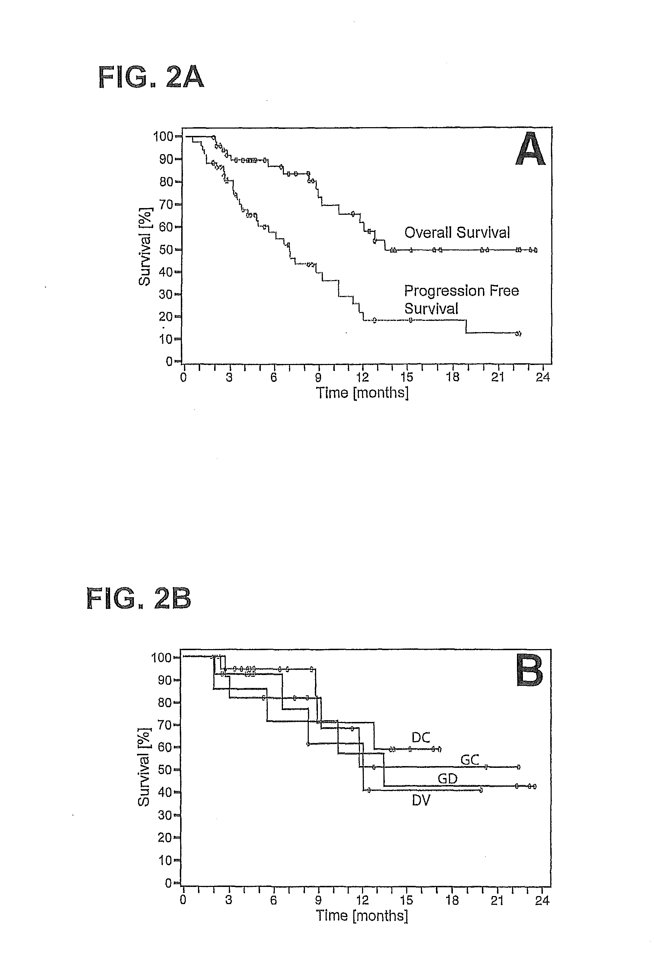

FIGs. 2A and 2B are graphs showing patient survival levels. FIG. 2A shows overall and progression-fiee survival of 53 patients with advanced NSCLC treated with chemotherapy based on the expression of the genes RRM1 and ERCC1. FIG. 2B shows overall survival by assigned chemotherapy. GC, gemcitabine and carboplatin; GD, gemcitabine and docetaxe1; DC, docetaxel and carboplatin; DV, docetaxel and vinorelbine.

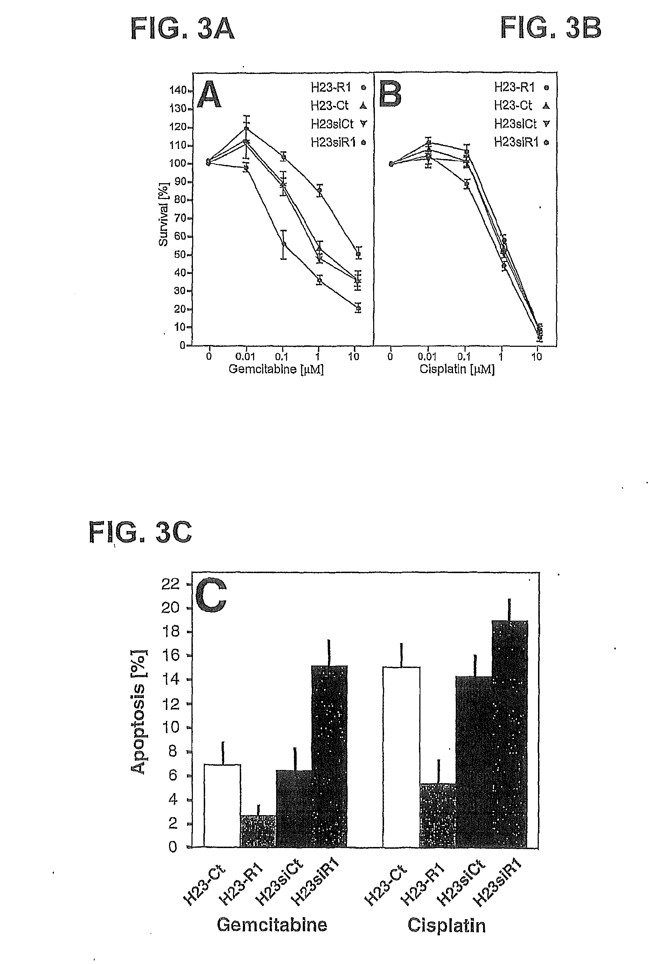

FIGs. 3A-3C are graphs showing effects of treatment with gemcitabine and cisplatin. FIGs. 3A and 3B show the *in vitro* efficacy of gemcitabine and cisplatin, respectively, in genetically modified cell lines. H23-R1 is a stable RRM1 overexpression cell line; H23siR1 is stably transformed with a sequence expressing an siRNA that targets RRM1; the control cell lines H23-Ct and H23siCt express RRM1 at levels similar to the parent H23 cell line. Cells were exposed to various concentrations for 72 hr, and viability was determined by MTS assay. Data are means +/- S.E. from three independent experiments. FIG. 3C shows apoptosis levels in cells exposed to 250 nM gemcitabine or 200 nM cisplatin for 6 hr. Percent apoptosis is a measure of the proportion of cells labeled with annexin V Data are means +/- S.E. from three independent experiments.

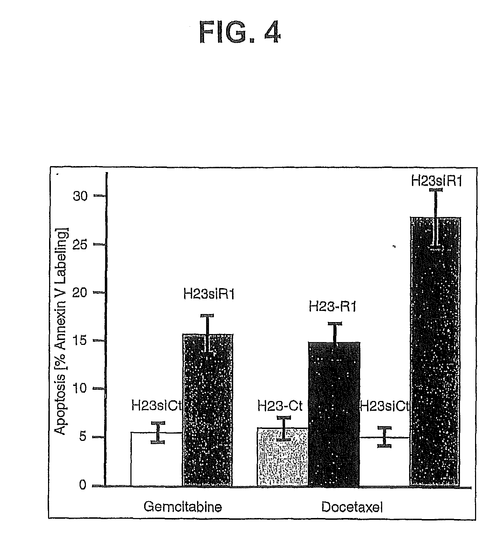

FIG. 4 is a graph showing induction of apoptosis by gemcitabine and docetaxel in H23siR1 and H23-R1 cell lines compared to control cell lines.

### DETAILED DESCRIPTION

The methods featured in the invention can be used to determine an appropriate therapy for a subject who has a proliferative disorder, such as an epithelial malignancy, or to predict or assess the efficacy of a composition containing a chemotherapeutic agent.

A "proliferative disorder," is a disorder characterized by irregularities in cell division. A cancer (*e*.*g*., a glioma, prostate cancer, melanoma, carcinoma, cervical cancer, breast cancer, colon cancer, or sarcoma) is an example of a proliferative disorder. Cells characteristic of proliferative disorders (*i*.*e*., "neoplastic cells" or "tumor cells") have the capacity for autonomous growth, *i*.*e*., an abnormal state or condition characterized by inappropriate proliferative growth of cell populations. A neoplastic cell or a tumor cell is a cell that proliferates at an abnormally high rate. A new growth comprising neoplastic cells is a neoplasm, also known as a "tumor." A tumor is an abnormal tissue growth, generally forming a distinct mass, that grows by cellular proliferation more rapidly than normal tissue. A tumor may show a partial or total lack of structural organization and functional coordination with normal tissue. As used herein, a tumor is intended to encompass hematopoietic tumors as well as solid tumors.

A tumor may be benign (benign tumor) or malignant (malignant tumor or cancer). Malignant tumors can be broadly classified into three major types. Malignant tumors arising from epithelial structures are called carcinomas; malignant tumors that originate from connective tissues such as muscle, cartilage, fat, or bone are called sarcomas; and malignant tumors affecting hematopoietic structure (structures pertaining to the formation of blood cells) including components of the immune system are called leukemias and lymphomas. Other tumors include, but are not limited to, neurofibromatoses.

Proliferative disorders include all types of cancerous growths or oncogenic processes, metastatic tissues or malignantly transformed cells, tissues, or organs, irrespective of histopathologic type or stage of invasiveness. Cancers include malignancies of various organ systems, such as the lung, breast, thyroid, lymphoid, gastrointestinal, and genito-urinary tract, as well as adenocarcinomas, which include malignancies such as most colon cancers, renal-cell carcinoma, prostate cancer and/or testicular tumors, non-small cell carcinoma of the lung, cancer of the small intestine and cancer of the esophagus. Carcinomas include malignancies of epithelial or endocrine tissues, such as respiratory system carcinomas, gastrointestinal system carcinomas, genitourinary system carcinomas, testicular carcinomas, breast carcinomas, prostatic carcinomas, endocrine system carcinomas, and melanomas. Other carcinomas include those forming from tissue of the cervix, lung, head and neck, colon and ovary. Cancers of the central nervous system include gliomas, (including astrocytomas, mixed oligoastrocytomas, glioblastoma multiform, ependymoma, and oligodendroglioma), meningiomas, pituitary tumors, hemangioblastomas, acoustic neuromas, pineal gland tumors, spinal cord tumors, hematopoietic tumors, and central nervous system lymphomas. Cancers affecting connective tissue, such as fat, muscle, blood vessels, deep skin tissues, nerves, bones, and cartilage are called sarcomas. Sarcomas include, for example, liposarcomas, leiomyosarcomas, rhabdomyosarcomas, synovial sarcomas, angiosarcomas, fibrosarcomas, neurofibrosarcomas, Gastrointestinal Stromal Tumors (GISTs), desmoid tumors, Ewing's sarcomas, osteosarcomas, and chondrosarcomas.

The methods described herein are particularly relevant for the treatment of humans having an epithelial malignancy, such as a lung cancer (*e*.*g*., non-small-cell lung cancer (NSCLC)), breast cancer, colorectal cancer, head and neck cancer, or ovarian cancer. Epithelial malignancies are cancers that affect epithelial tissues.

A "subject" as described herein can be any subject having a proliferative disorder. For example, the subject can be any mammal, such as a human, including a human cancer patient. Exemplary nonhuman mammals include a nonhuman primate (such as a monkey or ape), a mouse, rat, goat, cow, bull, pig, horse, sheep, wild boar, sea otter, cat, and dog.

An "antimetabolite" as used herein is a chemical with a similar structure to a substance (a metabolite) required for normal biochemical reactions, yet different enough to interfere with the normal functions of cells. Antimetabolites include purine and pyrimidine analogs that interfere with DNA synthesis. Exemplary antimetabolites include, *e*.*g*., aminopterin, 2-chlorodeoxyadenosine, cytosine arabinoside (ara C), cytarabine, fludarabine, fluorouracil (5-FU) (and its derivatives, which include capecitabine and tegafur), gemcitabine, methopterin, methotrexate, pemetrexed, raltitrexed, trimetrexate, 6-mcrcaptopurine, and 6-thioguanine.

An "antitubulin" as used herein refers to a chemotherapeutic agent that blocks cell division by inhibiting the mitotic spindle. Antibulin agents include, for example, the taxanes paclitaxel and docetaxel, and the vinca alkaloids vinorelbine, vincristine, vinblastine, vinflunine, and vindesine.

A "platinum-containing agent" as used herein includes chemotherapeutic agents that contain platinum. Platinum-containing agents cross-link with and alkylate DNA, winch results in the inhibition of DNA synthesis and transcription. The platinum-containing agents can act in any cell cycle, and consequently kill neoplastic as well as healthy dividing cells. Platinum-containing agents include, for example, cisplatin, carboplatin and oxaliplatin.

*Method of determining cancer therapy.* Methods of determining an appropriate cancer therapy include obtaining or providing a tumor sample from a patient, and determining the level of expression of RRM1 or ERCC1 in the patient. Any method can be used to obtain a tumor sample, such as a biopsy (*e*.*g*., core needle biopsy), and the tissue can be embedded in OCT^{®} (Optimal Tissue Cutting compound) for processing. For example, the tissue in OCT^{®} can be processed as frozen sections. Tumor cells can be collected, such as by laser capture microdissection (LCM), and gene expression can be assayed by, for example, reverse transcription coupled to polymerase chain reaction (RT-PCR) or Northern blot analysis to measure RNA levels, or by Western blot, to measure protein levels. In one exemplary approach, the level of RRM1 or ERCC1 expression is assayed by real-time quantitative RT-PCR. The level of expression of these genes can also be determined by immunohistochemistiy.

If intratumoral levels of RRM1 are low, it can be determined that a chemotherapy containing an antimetabolite, such as gemcitabine or pemetrexed, is appropriate. If intratumoral levels of RRM1 are high, it can be determined that a chemotherapy lacking an antimetabolite is appropriate. If intratumoral levels of ERCC1 are low, it can be determined that a chemotherapy containing a platinum-containing agent is appropriate. For example, if intratumoral levels of ERCC1 are low, a chemotherapy containing cisplatin, carboplatin or oxaliplatin can be determined to be appropriate. If intratumoral levels of ERCC1 are high, it can be determined that a chemotherapy lacking a platinum-containing agent is appropriate.

"Low" and "high" expression levels are relative values and are based on a comparison with those of a reference cohort. A "reference cohort," as used herein, is a sample cancer population from which RRM1 and/or ERCC1 expression data is collected. The expression level in a reference cohort is determined by measuring intratumoral gene expression levels in the sample population (see, *e*.*g.,* Rosell et al., Clin Cancer Res 10:1318-25, 2004; Lord et al., Clin Cancer Res 8:2286-2291, 2002; Bepler et al., J Clin Oncol 22:1878-85, 2004; and Simon et al., Chest 127:978-83, 2005). Typically, a tumor exhibits "low" RRM1 levels if the expression level is equal to or less than the median RRM1 expression level in the reference cohort, and the tumor exhibits "high" RRM1 levels if the expression level is greater than the median RRM1 expression level in the reference cohort, Similarly, a tumor exhibits "low" ERCC1 levels if the expression level is equal to or less than the median ERCC1 expression level in the reference cohort. "Low" and "high" expression levels are relative and can be established with each new reference group. In one alternative, the expression level determined to be predictive of a subject's response to a chemotherapy can be equal to or less than the expression level of the lowest third, or lowest quartile of a reference cohort, or the predictive expression level can be determined to be a level equal to or greater than the expression level of the highest third, or highest quartile of a reference cohort.

The samples from a reference cohort are taken from subjects of the same species (*e*.*g*., human subjects), and the tumors of a reference cohort are preferably of the same type (*e*.*g*., tumors of a NSCLC). For example, the tumors of a reference cohort can all be, for example, carcinomas, hematopoietic tumors, brain tumors, or sarcomas. In some embodiments, the tumors of a reference cohort can all be, for example, from a lung cancer, a breast cancer, a colorectal cancer, a head and neck cancer, or an ovarian cancer. The individual members of a reference cohort may also share other similarities, such as similarities in stage of disease, previous treatment regimens, lifestyle (*e*.*g*., smokers or nonsmokers, overweight or underweight), or other demographics (*e*.*g*., age, genetic disposition). For example, besides having the same type of tumor, patients in a reference cohort may not have received any previous systemic chemotherapy. A reference cohort should include gene expression analysis data from tumor samples from at least 10, 15, 20, 25, 30, 40, 50, 60,70, 80, 90, 100, 120, 140, 160, 180, or 200 subjects.

Gene expression levels in a reference cohort can be determined by any method, such as by quantitive RT-PCR, Northern blot anaylsis, Western blot analysis, or immunohistochemistry. Expression levels in a tumor sample from a test subject are determined in the same manner as expression levels in the reference cohort.

The tumor can be sampled for expression levels of RRM1 or ERCC1 or both, and an appropriate chemotherapy can be determined based on the observed expression levels. The chemotherapy can include a single agent or multiple chemotherapeutic agents (*e*.*g*., two, three, or more chemotherapeutic agents). For example, when intratumoral expression levels of RRM1 are determined to be low, an appropriate chemotherapy can be determined to be an antimetabolite alone. In one alternative, an appropriate chemotherapy can be determined to include an antimetabolite and a second agent, such as an antitubulin (*e*.*g*., a taxane or vinca alkaloid), an alkylating agent (*e.g.*, ifosfamide, cyclophosphamide, or other nitrogen-mustard derivatives), or a platinum-containing agent (*e*.*g*., cisplatin, carboplatin, or oxaliplatin). When intratumoral expression levels of RRM1 are determined to be high, an appropriate chemotherapy can be determined to include a platinum-containing agent, for example, but the appropriate chemotherapy should not include an antimetabolite.

In another example, if intratumoral expression levels of RRM1 are determined to be in the lowest third or quartile as compared to a reference cohort, or in the highest third or quartile as compared to a reference cohort, an appropriate chemotherapy can be determined to be an antitubulin agent alone. Alternatively, an appropriate chemotherapy can be determined to include an antitubulin agent, and a second agent, such as an antimetabolite or a platinum-containing agent. When intratumoral expression levels of RRM1 are determined to be in the middle 40-60% range as compared to a reference cohort, an appropriate chemotherapy can be determined to include one or more of an antimetabolite or a platinum-containing agent, but the appropriate chemotherapy should not include an antitubulin agent.

In another example, if intratumoral expression levels of ERCC1 are determined to be low, an appropriate chemotherapy can be determined to be a platinum-containing agent alone. Alternatively, an appropriate chemotherapy can be determined to include a platinum-containing agent, and a second agent, such as an antimetabolite or an antitubulin. When intratumoral expression levels of ERCC1 are determined to be high, an appropriate chemotherapy can be determined to include one or more of an antimetabolite or an antitubulin, but the appropriate chemotherapy should not include a platinum-containing agent.

In yet another example, intratumoral expression levels of both RRM1 and ERCC1 are determined, and an appropriate chemotherapeutic agent is determined based on the expression level of both genes. For example, if RRM1 and ERCC1 expression levels are both determined to be low, an appropriate chemotherapy can be determined to include an antimetabolite, and a platinum-containing agent, such as carboplatin, cisplatin, or oxaliplatin. If RRM1 levels are determined to be low and ERCC1 levels are determined to be high, an appropriate chemotherapy can be determined to include an antimetabolite, and an optional second agent, such as an antitubulin. Such a chemotherapeutic composition should not include a platinum-containing agent. If RRM1 levels are determined to be high and ERCC1 levels are determined to be low, an appropriate chemotherapy can be determined to include a platinum-containing agent and an optional second agent, such as an antitubulin or an alkyalting agent. Such a chemotherapeutic composition should not include an antimetabolite. If RRM1 and BRCC1 levels are both determined to be high, an appropriate chemotherapy can be determined to include an antitubulin. The chemotherapy should not include an antimetabolite or a platinum-containing agent.

In yet another example, if ERCC1 expression levels are low, and RRM1 expression levels fall within the lowest third or quartile, or highest third or quartile as compared to a reference cohort, then an appropriate chemotherapy can be determined to include an antitubulin, such as docetaxel or vinorelbine, and a platinum-containing agent. If ERCC1 levels are determined to be high, and RRM1 expression levels fall within the lowest third or quartile, or highest third or quartile as compared to a reference cohort, then an appropriate chemotherapy can be determined to include an antitubulin, and an optional second agent, such as an antimetabolite. Such a chemotherapeutic composition should not include a platinum-containing agent. If ERCC1 levels are determined to be low, and RRM1 expression levels fall within the middle 40-60% as compared to a reference cohort, then an appropriate chemotherapy can be determined to include a platinum-containing agent and an optional second agent, such as an antimetabolite or an alkyalting agent. Such a chemotherapeutic composition should not include an antitubulin. If ERCC1 levels are determined to be high and RRM1 expression levels fall within the middle 40-60% as compared to a reference cohort, then an appropriate chemotherapy can be determined to include an antimetabolite. The chemotherapy should not include an antitubulin or a platinum-containing agent.

Other chemotherapeutic agents can be administered with the antimetabolite, antitubulin, or platinum-containing agent, or *in lieu* of an antimetabolite, antitubulin, or platinum-containing agent according to RRM1 and ERCC1 expression levels as described herein. Other chemotherapeutic agents include, for example, L-asparaginase, bicalutamide, bleomycin, camptothecin (CPT-11), carminomycin, cyclophosphamide, cytosine arabinoside, dacarbazine, dactinomycin, doxorubicin, daunorubicin, ecteinascidin 743, estramustine, etoposide, etoposide phosphate, epothilone, flutamide, FK506, hexamethyl melamine, idatrexate, leflunimide, leuprolide, leurosidine, leurosine, melphalan, mitomycin C, mycophenolate mofetil, plicamycin, podophyllotoxin, porfiromycin, ranpirnase, rapamycin, topotecan, teniposide, and thiotepa.

A subject who is administered a chemotherapy according to intratumoral RRM1 or ERCC1 expression levels can further be administered a radiation therapy, immunotherapy or surgery.

A chemotherapy can be administered to a subject using conventional dosing regimens. The appropriate dosage will depend on the particular chemotherapeutic agents determined to be appropriate for the subject based on RRM1 and/ox ERCC1 expression levels as described herein.

Chemotherapy can be administered by standard methods, including orally, such as in the form of a pill, intravenously, by injection into a body cavity (such as the bladder), intramuscularly, or intrathecally. A chemotherapy regimen can be delivered as a continuous regimen, *e*.*g*., intravenously, orally, or in a body cavity. A chemotherapy regimen can be delivered in a cycle including the day or days the drug is administered followed by a rest and recovery period. The recovery period can last for one, two, three, or four weeks or more, and then the cycle can be repeated. A course of chemotherapy can include at least two to 12 cycles (*e*,*g*., three, four, five, six, seven, ten or twelve cycles).

Gene expression data obtained from the methods featured herein can be combined with information from a patient's medical records, including demographic data; vital status; education; history of alcohol, tobacco and drug abuse; medical history; and documented treatment to adjust conclusions relating to the prognosis of a proliferative disorder following administration of a chemotherapy designed as described above.

Upon administration of a chemotherapy according to the intratumoral RRM1 or ERCC1 expression levels, a patient can be monitored for a response to the therapy. For example, tumor measurements can be taken before and after administration of the chemotherapy to monitor disease progression. If tumor size decreases, the disease can be determined to be in remission, or regressing towards remission. A partial decrease in tumor size can indicate a disease in partial remission, and if the tumor completely disappears, the disease can be said to be in complete remission. If tumor size increases, the disease can be determined to be progressing. If tumor size does not change following administration of the chemotherapy, the disease can be categorized as stable.

A patient can also be assessed according to the stage of cancer. For example, the patient can be assessed according to the TNM staging system. "T" describes the size of the tumor and whether it has invaded nearby tissue, "N" describes any lymph nodes that are involved, and "M" describes metastasis (spread of cancer from one body part to another). According to the TNM scale, stage 0 indicates carcinoma *in situ,* which is an early cancer present only in the layer of cells in which it began. Stages I, II, III, and IV indicate progressively worsening disease states. Higher stages indicate more extensive disease as evidenced by greater tumor size and/or spread of the cancer to nearby lymph nodes and/or organs adjacent to the primary tumor. In stage IV, the tumor has spread to at least one other organ.

A subject can also be assessed according to his physical condition, with attention to factors such as weight loss, pleural effusion, and other symptoms related to the cancer. For example, symptoms of lung cancer, including small-cell and non-small cell lung carcinoma include persistent cough, sputum streaked with blood, chest pain, and recurring pneumonia or bronchitis.

The methods featured in the invention can be performed on any subject of any age, including a fetus (*e*.*g*., *in utero*), infant, toddler, adolescent, adult, or elderly human.

*Methods of predicting efficacy and screening methods.* The invention also features methods of assessing or predicting the efficacy of a composition containing a chemotherapeutic agent. The methods employ at least two cell lines and a composition containing one or more chemotherapeutic agents. The cell lines differ in their level of expression of RRM1 or ERCC1. For example, one cell line expresses a lower level of RRM1 than a standard control cell line, or one cell line expresses a higher level of RRM1 than a standard control cell line. The higher-expression cell line preferably expresses at least about 20%, 40%, 60%, 80%, 100%, 200% or 300% more RRM1 or ERCC1 than one lower-expression cell line.

Any manner of causing increased or decreased expression of RRM1 or ERCC1 can be utilized. For example, a cell line expressing a lower level of RRM1 can be engineered to express an siRNA or antisense RNA that causes the lower level of expression. Alternatively, RRM1 expression can be placed under control of a regulatable promoter, such as a tetracycline-, IPTG- or eedysone-responsive promoter. RRM1 expression may be lower than expression in a parent strain, or expression may be completely absent prior to induction. Cells expressing high levels of RRM1 can contain an RRM1 gene under control of a constitutive promoter that expresses RRM1 at a higher level than the endogenous RRM1 promoter, or RRM1 can be expressed from an inducible promoter, such as a tetracycline- or IPTG-responsive promoter, such that induction drives expression to a greater level than that in the parent strain. An exogenous sequence that drives a higher level of RRM1 expression, or directs a lower level of expression can be stably integrated into the genome of the parent strain, or can be transiently transfected into the parent strain.

Cells that express high and low levels of RRM1 or ERCC1 are contacted with a candidate chemotherapeutic agent or a combination of chemotherapeutic agents (*e*.*g*., 2, 3, or 4 chemotherapeutic agents), and the cells are monitored for an increased sensitivity or resistance to the chemotherapeutic agent or agents as compared to a control strain. An agent, or combination of agents, that causes an increased sensitivity to one cell line over a control cell line can be identified as a candidate therapeutic agent for a patient who has a tumor expressing the corresponding level of RRM1 or ERCC1.

For example, if cells expressing low levels of RRM1 (*i*.*e*., lower levels than a control parent strain) are more sensitive to a chemotherapeutic agent than the cells of the control strain, then the agent is a candidate therapeutic agent for the treatment of a patient with a tumor expressing low levels of RRM1. If cells expressing high levels of RRM1 (*i*.*e*., higher levels than a control parent strain) are more sensitive to a chemotherapeutic agent than the cells of the control strain, then the agent is a candidate therapeutic agent for the treatment of a patient with a tumor expressing high levels of RRM1.

In another example, if cells expressing low levels of ERCC1 (*i*.*e*., lower levels than a control parent strain) are more sensitive to a chemotherapeutic agent than the cells of the control strain, then the agent is a candidate therapeutic agent for the treatment of a patient with a tumor expressing low levels of ERCC1. If cells expressing high levels of ERCC1 (*i*.*e*., higher levels than a control parent strain) are more sensitive to a chemotherapeutic agent than the cells of the control strain, then the agent is a candidate therapeutic agent for the treatment of a patient with a tumor expressing high levels of ERCC1.

The methods described herein can be used in screening assays to identify agents that are candidates for the treatment of tumors expressing high or low levels of RRM1 or ERCC1. Cell lines such as those described above can be contacted with a panel of agents (*e*.*g*., small molecule drugs, nucleic acids, or polypeptides) to identify agents that cause increased sensitivity of cells expressing low or high levels of RRM1.

Agents identified in the above screening methods, or agents or combinations of agents identified by the above methods as candidate chemotherapies can be tested in animal models before being tested in humans. For example, the therapies can be tested for the ability to reduce tumor size in mice or primate models, before testing in humans.

*Kits.* Reagents, tools, and/or instructions for performing the methods described herein can be provided in a kit. For example, the kit can contain reagents, tools, and instructions for determining an appropriate therapy for a cancer patient. Such a kit can include reagents for collecting a tissue sample from a patient, such as by biopsy, and reagents for processing the tissue. The kit can also include one or more reagents for performing a gene expression analysis, such as reagents for performing RT-PCR, Northern blot, Western blot analysis, or immunohistochemistry to determine RRM1 and/or ERCC1 expression levels in a tumor sample of a human. For example, primers for performing RT-PCR, probes for performing Northern blot analyses, and/or antibodies for performing Western blot and immunohistochemistry analyses can be included in such kits. Appropriate buffers for the assays can also be included. Detection reagents required for any of these assays can also be included.

The kits featured herein can also include an instruction sheet describing how to perform the assays for measuring gene expression. The instruction sheet can also include instructions for how to determine a reference cohort, including how to determine RRM1 and/or ERCC1 expression levels in the reference cohort and how to assemble the expression data to establish a reference for comparison to a test subject. The instruction sheet can also include instructions for assaying gene expression in a test subject and for comparing the expression level with the expression in the reference cohort to subsequently determine the appropriate chemotherapy for the test patient. Methods for determining the appropriate chemotherapy are described above and can be described in detail in the instruction sheet.

In another example, a kit featured in the invention can contain reagents, tools, and instructions for predicting the efficacy of a candidate chemotherapeutic agent based on RRM1 or ERCC1 expression levels. Such a kit can include vectors for modulating RRM1 or ERCC1 expression levels in a cell, and reagents for monitoring cell phenotype, such as reagents for detecting apoptosis. Reagents for determining the expression levels of RRM1 and ERCC1 in the tissue samples can also be included as described above.

Informational material included in the kits can be descriptive, instructional, marketing or other material that relates to the methods described herein and/or the use of the reagents for the methods described herein. For example, the informational material of the kit can contain contact information, *e*.*g*., a physical address, email address, website, or telephone number, where a user of the kit can obtain substantive information about performing a gene expression analysis and interpreting the results, particularly as they apply to a human's likelihood of having a positive response to a specific chemotherapy.

A kit can contain separate containers, dividers or compartments for the reagents and informational material. A container can be labeled for use for the determination of RRM1 and ERCC1 gene expression levels and the subsequent determination of an appropriate chemotherapy for the human.

The informational material of the kits is not limited in its form. In many cases, the informational material, *e*.*g*., instructions, is provided in printed matter, *e*.*g*., a printed text, drawing, and/or photograph, *e*.*g*., a label or printed sheet. However, the informational material can also be provided in other formats, such as Braille, computer readable material, video recording, or audio recording. Of course, the informational material can also be provided in any combination of formats.

The invention is further illustrated by the following examples, which should not be construed as further limiting.

### EXAMPLES

**Example 1: Reduced expression of RRM1 correlates with increased sensitivity to gemcitabine.** Stable, genetically modified cell lines with increased and decreased RRM1 expression and corresponding controls were generated (Gautam et al., Oncogene 22:2135-42, 2003). H23, originating from a lung adenocarcinoma, was used as the parent cell line (Carney et al., Cancer Res 45:2913-23, 1985). H23siR1 was generated by stable transfection of NCI-H23 with a pSUPER-siRRM1 construct. These cells express an siRNA that targets RRM1, thereby causing decreased expression of RRM1. To generate this construct, the pSUPER-GFP/neo vector (OligoEngine, Seattle, WA) was digested with BglII and HindIII and the annealed oligonucleotides (5'-GATCCCCgacgctagagcggtcttatTTCAAGAGAataagaccgctctagcgtcTTTTTGGAAA-3'(SEQ ID NO:1) and 5'- AGCTTTTCCAAAAAgacgctagagcggtcttatTC TCTTGAAataagaccgctctagcgtcGGG -3' (SEQ ID NO:2)) were ligated into the vector. The 19 nucleotide RRM1 target sequences are denoted by lower-case letters. The control H23siCt cell line was generated which expressed an siRNA targeting the sequence GCTAATAGCGCGGAGTCTT (SEQ ID NO:3). This sequence has no similarity to any known gene. H23-R1 is a stable RRM1 overexpressing cell line, and H23-Ct is its corresponding control. Both were generated by transfection with full-length RRM1 cDNA cloned into the expression plasmid pCMV-Tag2 (Stratagene, La Jolla, CA) as previously described (Gautam et al., Oncogene 22:2135-42, 2003). The level of RRM1 expression in these cell lines was assessed by real-time quantitative RT-PCR and Western blot analysis.

A modified MTT assay (MTS assay, Promega, Madison, WI) was used to assess the impact of RRM1 expression on therapeutic efficacy of gemcitabine and platinum-containing agents *in vitro.* This MTS assay utilized 3-(4,5-dimethylthiazol-2-yl)-5-(3-carboxylnethoxyphenyl)-2-(4-sulfophenyl)-2H-tetrazolium as the reagent. The assay was performed by seeding 4,000 cells/well in 96 well flat-bottom plates. H23-Ct, H23-R1, H23siR1, H23siCt cells were continuously exposed to drug for 72 or 96 hr. After 3 hr of incubation at 37°C, the absorbance was measured at 490 nm in a microplate reader (Benchmark Plus, Bio-Rad, Hercules, CA). All experiments were repeated three times. The IC₅₀ (*i*.*e*., the drug concentration that inhibited cell growth by 50%) was calculated as: [(mean absorbance in triplicate wells containing the drug - mean absorbance in triplicate blank wells) / (mean absorbance in triplicate drug-free wells - mean absorbance in triplicate blank wells)] x 100.

H23-R1 had a 2.5 to 3.5-fold increased RRM1 expression compared to H23 (Gautam et al., Oncogene 22:2135-42, 2003). H23siR1 had a 5-fold reduced RRM1 expression. The expression levels of RRM1 and control genes were determined by real-time quantitative RT-PCR and Western blotting. The expression of RRM2, the catalytic ribonucleotide reductase subunit, was unaffected. The sensitivity of these cell lines to gemcitabine, cisplatin, and carboplatin was compared to transfected control cell lines (H23-Ct and H23siCt). Increased RRM1 expression resulted in resistance to gemcitabine (Table 1, see FIG. 3A and FIG. 3C). The gemcitabine IC₅₀ of H23-R1 was 8-fold higher than the IC₅₀ of H23-Ct. Reduced RRM1 expression increased sensitivity to gemcitabine. The gemcitabine IC₅₀ of H23siR1 was 10-fold lower than the IC₅₀ of H23siCt. The response of the parental cell line (H23) to gemcitabine was similar to the response of H23-Ct and H23siCt. There was a similar relationship between RRM1 expression and cytotoxicity response to cisplatin (Table 1) and carboplatin although to a lesser degree. H23-R1 was 1.2-1.5-fold more resistant and H23siR1 was 1.1-1.3-fold more sensitive to platinum-containing agents than the corresponding control cell lines.

**Table 1. In Vitro Response of Cell Lines with Modified RRM1 Expression Levels.**

| Cell line | Relative *RRM1* Expression by real-time RT-PCR | Gemcitabine IC₅₀ +/- SD [nM] | Cisplatin IC₅₀+/-SD [nM] | Carboplatin IC₅₀ +/- SD [nM] |
|---|---|---|---|---|
| H23 | Control | 500 +/- 9 | 1,200 +/- 140 | 12,000 +/- 1,732 |
| H23-R1 | 3-fold up | 6,000 +/-107 | 1,500 +/- 85 | 20,000 +/- 0 |
| H23-Ct | No change | 750 +/- 12 | 1,250 +/- 50 | 15,000 +/- 577 |
| H23siR1 | 5-fold down | 100 +/- 29 | 950 +/- 7 | 10,000 +/- 1,000 |
| H23siCt | No change | 1,015 +/- 140 | 1,000 +/- 210 | 13,000 +/- 1,414 |

To determine drug-induced *in vitro* apoptosis, 3x10⁵ cells/well were seeded in six-well plates and allowed to attach overnight. Chemotherapeutic agents were added to the culture at the indicated concentrations. After 2, 4, and 6 hours of treatment annexin V labeling of the cells was performed as recommended by the manufacturer (BD Pharmingen, San Diego, CA). The percentage of labeled cells was determined by flow cytometry (FACScalibur, Becton Dickinson, Franklin Lakes, NJ). The annexin V labeling experiments further revealed an inverse relationship between drug induced cell death and RRM1 expression (see FIGs. 3A-3C). The proportion of cells labeled after 6 h of treatment with 250 nM gemcitabine was 6.6-6.9% in control cell lines, 2.6% in H23-R1, and 15.2% in H23siR1. Cisplatin exposure for 6 h at 200 nM yielded 14.5-15.1% apoptotic cells in H23-Ct and H23siCt, 5.5% apoptotic cells in H23-R1, and 19.0% apoptotic cells in H23siR1.

**Example 2. Clinical trials revealed an inverse correlation in RRM1 expression and response to gemcitabine-based chemotherapy.** To prospectively address the hypothesis that intratumoral RRM1 expression is predictive of response to gemcitabine-based chemotherapy, we conducted a clinical trial in patients with locally advanced non-small-cell lung cancer (NSCLC). The institutional review board approved the clinical trials, and all subjects provided written informed consent.

The impact of tumoral RRM1 expression on the efficacy of two cycles of first line gemeitabine-based chemotherapy was assessed. Patients with locally advanced and inoperable NSCLC received gemcitabine and carboplatin as induction therapy (IndGC). Double agent chemotherapy was chosen because single agent therapy is deemed inadequate for previously untreated patients with any stage of NSCLC (National Comprehensive Cancer Network guidelines or NSCLC treatment). Trial eligibility included histologically confirmed NSCLC, inoperable stage IIIA or IIIB disease (patients with cytologically positive pleural effusion were excluded; an exemption was made for one patient with stage IIB disease), measurable disease by RECIST (Therasse et al., J Natl Cancer Inst 92:205-16, 2000), no prior systemic chemotherapy or thoracic radiation, performance status zero or one by Eastern Cooperative Oncology Group criteria, absence of weight loss (<5% in the three months preceding diagnosis), and adequate bone marrow, liver, and kidney function.

All patients were staged with computed tomography of chest and upper abdomen (CT), whole body FDG positron emission tomography (PET), and magnetic resonance imaging of the brain. IndGC consisted of two 28-day cycles of Gemcitabine, 1,000 mg/m², given on days 1 and 8, and carboplatin, AUC 5, given on day 1. CT and PET were repeated in week 8, and disease response was assessed by unidimensional measurement of lesion on CT according to RECIST. Thereafter, patients received concurrent radiation and chemotherapy for definitive treatment of their disease. The study required the collection of tumor specimens specifically for molecular analyses prior to therapy, which was performed by core needle biopsy that produced a tissue specimen of 0.8 mm diameter. Specimens were immediately frozen in liquid nitrogen, embedded in OCT^{®}, and processed as frozen sections. Tumor cells were collected by laser capture microdissection (LCM), and RNA was extracted.

For gene expression analysis, frozen tissue samples, embedded in OCT^{®}, were cut in 5-7 µM sections. Tumor cells were collected by LCM using the Arcturus system (60 mW, 1.5 msec, intensity 100, spot size ∼20 µm), and total RNA was extracted using a commercially available method (PicoPure RNA isolation kit, #KIT0204, Arcturus, Mountain View, CA). Complementary DNA was generated with Superscript II and oligo-dT (Invitrogen, Carlsbad, CA). Real-time quantitative PCR gene analysis was performed in triplicate per sample in 96-well plates (ABI prism 7700, Perkin-Elmer, Foster City, CA). Each plate contained a serial dilution of reference cDNA for standard curve determination and negative controls without template. Primers and probes designed for RRM1 and ERCC1 expression analysis were described previously (Bepler et al., J Clin Oncol 22:1878-85, 2004; Simon et. al., Chest 127:978-83, 2005). Commercially available primers and probes were used for expression analysis of the housekeeping gene 18S rRNA (Perkin-Elmer, #4310893E-0203015), which was used as an internal reference standard. The relative amount of target RNA in a sample was determined by comparing the threshold cycle with the standard curve, and the standardized amount was then determined by dividing the target amount by the 18S rRNA amount.

Unidimensional tumor measurements were obtained before and after chemotherapy, and disease response was recorded as the percent change after treatment compared to before treatment and also categorized as complete remission (CR), partial remission (PR), stable disease (SD), and progressive disease (PD) according to RECIST. At least one and up to six separate cancer lesions were measured in greatest diameter using images obtained with intravenous contrast on a multichannel helical CT scanner. Measurements were performed on a picture-archive-comznunication system workstation (Siemens MagicView 1000), and they were repeated at 6-8 weekly intervals. The percentage of change of the sum of tumor diameters comparing the post treatment with the pretreatment measurements was calculated using the formula 1-(SumCTpost / SumCTpre). A positive value indicated tumor shrinkage and a negative value tumor growth. The appearance of a new and previously not observed tumor lesions on imaging studies or physical exams were coded as disease progression. Overall survival (OS) was recorded as the time elapsed from the date of first treatment to the date of death. Pxogression-free survival (PFS) was recorded as the time elapsed from the date of first treatment to the date of first evidence for disease progression or death. Patients without an event were censored at the last date of follow-up (March 24, 2006).

Between November 2003 and January 2006, 40 eligible patients were enrolled. Three patients withdrew consent prior to initiation of therapy because they desired treatment closer to home. In 28 patients, the biopsies were performed to obtain tissue for molecular studies only, in 9 patients they were also used to establish a diagnosis. In one patient, a pneumothorax developed that required chest tube placement. In two patients tumor collection did not yield sufficient material for gene expression analysis. From the remaining 35 patients, between 30 and 4,500 tumor cells were collected by LCM. All 35 patients completed IndGC, and disease response ranged from a 9% increase to a 100% decrease in tumor diameters. Twenty patients had SD, fourteen had PR, and one had CR. Patients' clinical characteristics are summarized in Table 2.

**Table 2. RRM1 and ERCC1 Expression and Patients' Characteristics in Both Trials**

| | Prospective Trial for Gene Expression/Disease Response Assessment in Locally Advanced NSCLC | Prospective Trial with Treatment Selection Based on Gene Expression in Advanced Metastatic NSCLC |
|---|---|---|
| Patients Enrolled [N] | 40 | 69 |
| Patients With Successful Gene Analysis [N] | 35 | 53 |
| Tumor Cells used for Gene Analysis [N] | 30 - 4,500 | 180 - 3,000 |
| *RRM1* Expression (median, range) | 2.85, 0.18 -129.3 | 12.1, 0.0 -1,637.3 |
| *ERCC1* Expression (median, range) | 6.70, 0.30 - 741.1 | 12.4, 0.9 - 8,102.8 |
| Disease Response [N] | | |
| CR | 1 (3%) | 0 (0%) |
| PR | 14 (40%) | 22 (42%) |
| SD | 20 (57%) | 24 (46%) |
| PD | 0 (0%) | 6 (12%) |
| Not evaluable | 0 | 1 |
| Tumor Histology [N] | | |
| Adenocarcinoma | 11 (31%) | 33 (62%) |
| Squamous Cell Carcinoma | 11(31%) | 2 (4%) |
| Large Cell or Unspecified NSCLC | 13 (37%) | 18 (34%) |
| Tumor Stage [N] | | |
| IIB | 1 (3%) | NA |
| IIIA | 18 (51%) | NA |
| IIIB (including non-malignant pleural effusion) | 16 (46%) | NA |
| IIIB (malignant pleural effusion) | NA | 1 (2%) |
| IV | NA | 52 (98%) |
| Gender male/female [N] | 18 / 17 | 31 / 22 |
| Age median (range) [Years] | 63 (47 - 87) | 63 (38 - 78) |
| Smoking Status s [N] | | |
| Life-Time Never Smoker | 2 (6%) | 6(11%) |
| Quit (>1 year) | 20 (57%) | 35 (66%) |
| Active | 13 (37%) | 12 (23%) |
| ECOG Performance Status [N] | | |
| Zero | 14 (40%) | 25 (47%) |
| One | 21 (60%) | 28 (53%) |
| Weight Loss >/= 5% in 3 months [N] | | |
| Absent | 34 (97%) | 49 (92%) |
| Present | 1 (3%) | 4 (8%) |

| | | |
|---|---|---|
| RRM1 and ERCC1 expression values are normalized according to the expression of the house-keeping gene 18S rRNA. | | |

RRM1 expression ranged from 0.18 to 129.3. There was a significant (p=0.002) inverse correlation (r=-0.498, Speannan correlation coefficient) between RRM1 expression and the magnitude of disease response (FIG. 1A). When grouping patients into those with response (CR/PR) and without response (SD), RRM1 expression was significantly (p=0.03, Chi-square test) associated with response, where patients with high tumoral expression of RRM1 were less likely to respond to IndGC than those with low levels of expression. ERCC1 expression was also inversely correlated (r=-0.283, p=0.099) with the magnitude of response (FIG. 1B) but not with the response classification (p=0.32) to IndGC (see Table 3). There was no significant association between gene expression and the number of tumor cells collected, patients' age or gender, tumor histopathology, and smoking status.

**Table 3. RRM1 and ERCC1 Expression and Association With Response to Gemcitabine and Carboplatin in Patients With Locally Advanced NSCLC.**

| | RRM1¹ | ERCC1¹ | *Disease Response²* |
|---|---|---|---|
| All Patients (N=35) | 2.85 | 6.70 | 23% |
| Median (Range) | (0.18 - 129.3) | (0.30 - 741.1) | (-9% - +100%) |
| Correlation with Response³ (p-value) | *r* = (-)0.498 | *r* = (-)0.283 | |
| | *p* = 0.002 | *p* = 0.099 | |
| Patients with PR/CR (N=15) | 1.84 | 3.79 | 44% |
| Median (Range) | (0.18 - 9.94) | (1.17 - 49.79) | (+30% - +100%) |
| Patients with SD (N=20) | 13.06 | 10.38 | 12% |
| Median (Range) | (0.87 - 129.3) | (0.30 - 741.1) | (-9% - +29%) |

| | | | |
|---|---|---|---|
| ¹Values given are corrected for expression of the house-keeping gene 18S rRNA; ²positive values indicate tumor reduction and negative values indicate tumor growth; ³Spearman correlation coefficient. | | | |

Statistical analyses included the following. Correlation coefficients between gene expression and the continuous variables tumor response, number of cells analyzed, and patient's age were calculated according to Spearman. Cox's Proportional Hazards analysis was used to assess the impact of gene expression on survival. The pooled t-test was used to test for significance between gene expression and gender or other dichotomous patient variables. The one way ANOVA test was used to test for significance between gene expression and patients' smoking status or other non-continuous patient variables with more than 2 values. OS and PFS probabilities were estimated using the Kaplan-Meier method. The Log-Rank test was used to determine the level of significance between survival curves.

### Example 3. Clinical trials revealed improved patient response and when chemotherapy was administered according to RRM1 and ERCC1 expression levels.

To test whether selection of chemotherapy based on gene expression would improve patient survival, we conducted a phase II single-institution treatment trial in patients with advanced and incurable NSCLC. In this study the decision on a double-agent chemotherapy regimen was based on the expression of the genes RRM1 and ERCC1. Trial eligibility included histologically confirmed NSCLC, stage IV or IIIB (with malignant pleural effusion) disease, measurable disease by RECIST (Therasse et al., J Natl Cancer Inst 92:205-16, 2000), no prior systemic chemotherapy in the three years preceding the diagnosis of advanced stage NSCLC, performance status zero or one by Eastern Cooperative Oncology Group criteria, age >18, and adequate bone marrow, liver, and kidney function. Patients with stable brain metastases were allowed to enroll. Prior radiation was allowed, provided it ended at least 3 weeks before initiation of study-selected chemotherapy, that the patient had recovered from radiotherapeutic toxicity, and that at least one measurable target lesion was outside the radiation field.

A core needle biopsy was required for tumor collection. Specimens were immediately frozen, sectioned, and subjected to LCM for tumor cell collection. The level of expression for RRM1 and ERCC1 was determined by real-time quantitative RT-PCR with custom-designed and validated primers and probes (Bepler et al., J Clin Oncol 22:1878-85, 2004; Simon et al., Chest 127:978-83, 2005). Decisions regarding chemotherapy agents were based on gene expression levels. Gemcitabine was given if RRM1 expression was equal to or below 16.5, and carboplatin was given if ERCC1 expression was equal to or below 8.7. These levels were selected based on our experience with expression analysis in patients with NSCLC with the goal to achieve values close to the expected cohort median (Rosell et al., Clin Cancer Res 10:1318-25, 2004; Lord et al., Clin Cancer Res 8:2286-2291, 2002; Bepler et al., J Clin Oncol 22:1878-85, 2004; Simon et al., Chest 127:978-83, 2005).

The actual medians in this patient cohort were similar (median RRM1 12.1, range 0.0 - 1,637.3; median ERCC1 12.4, range 0.9 - 8,102.8). Thus, the treatment for patients consisted of gemcitabine and carboplatin (GC) if RRM1 and ERCC1 were below the chosen values, gemcitabine and docetaxel (GD) if RRM1 was below and ERCC1 above the values, docetaxel and carboplatin (DC) if RRM1 was above and ERCC1 below the values, and docetaxel and vinorelbine (DV) if RRM1 and ERCC1 were above the values.

From February 2004 to December 2005, 69 eligible patients were enrolled. Eight withdrew consent (5 after the biopsy was done), and one was removed prior to therapy because of transportation problems. Gene expression analysis failed in 5 patients, and two patients never received the assigned treatment because of natural disasters in Florida during the summer of 2004 (Table 2). Of the 53 patients that were treated with the assigned therapy, 12 received GC, 20 GD, 7 DC, and 14 DV.

Drug delivery doses were as follows. Gemcitabine, 1,250 mg/m² on days 1 and 8, and carboplatin, AUC 5 on day 1, were given every 21 days. Gemcitabine, 1,250 mg/m² on days 1 and 8, and docetaxel, 40 mg/m² on days 1 and 8, were given every 21 days. Docetaxel, 75 mg/m² on day 1, and carboplatin, AUC 5 on day 1, were given every 21 days. Vinorelbine, 45 mg/m² on days 1 and 15, and docetaxel, 60 mg/m² on days 1 and 15, were given every 28 days. CT scans were done prior to therapy and after every two cycles. They were used to measure and record unidimensional disease response. Treatment was given until disease progression as defined by RECIST or for a total of 6 cycles. Further therapy was at the discretion of the treating physician. The best response to therapy was recorded for each patient and defined as the maximal tumor shrinkage as recorded by CT after cycles 2, 4, or 6. After completion of the study-selected chemotherapy, patients were followed at least every 3 months with CT scans for determination of disease status. Disease response, overall survival (OS), and progression-free survival (PFS) were determined and recorded as described above.

The best treatment response was PR in 22 patients (42%, 95% confidence interval (CI) 29-57%), SD in 24 patients (46%, 95% CI 32-61%), and PD in 6 patients (12%, 95% CI 4-23%, four developed new metastases, two had a greater than 20% increase in tumor diameters). Thus, the disease control rate (PR/SD) was 88.5% (95% CI 76.6-95.6%). One patient was not evaluable. He was unable to complete the first cycle of therapy because of drug-related hepatotoxicity.

The 6-months and 12-months OS rates were 87% (95% CI, 73-94%) and 62% (95% CI, 43-77%). The 6-months and 12-months PFS rates were 60% (95% CI, 45-73%) and 18% (95% CI, 7-33%) (FIG. 2A and Table 4).

**Table 4. Overall and Progression-Free Survival in Patients With Advanced NSCLC Treated Based on RRM1 and ERCC1 Expression and Comparison with Other Trials.**

| | This Phase II Study MCC-13208 | | Our Previous Phase II Study, MCC-12621¹ | | Randomized Phase III Study, ECOG-1594² | |
|---|---|---|---|---|---|---|
| | OS (95% CI) | PFS (95% CI) | OS (95% CI) | PFS (95% CI) | OS (95% CI) | PFS (95% CI) |
| 3 months | 90% (77-96%) | 81% (67-89%) | 75% (59-86%) | 65% (48-78%) | 82% (80-84%) | NA³ |
| 6 months | 87% (73-94%) | 60% (45-73%) | 58% (41-71 %) | 38% (23-52%) | 58% (63-74%) | NA |
| 9 months | 73% (55-85%) | 40% (24-55%) | 40% (25-55%) | 28% (15-42%) | 44% (41-46%) | NA |
| 12 months | 62% (43-77%) | 18% (7-33%) | 38% (23-52%) | 10% (3-21%) | 33% (31-36%) | NA |
| Response Rate⁴ | 42% (22/52) | | 24% (9/38) | | 19% (229/1207) | |
| Median OS Time | 13.4 months | | 6.7 months | | 8.0 months | |
| Median PFS Time | 7.0 months | | 4.9 months | | 3.7 months⁵ | |

| | | | | | | |
|---|---|---|---|---|---|---|
| ¹in Oncology 68: 382-390,2005; ²in N Engl J Med 346: 92-98, 2002; ³NA, not available; ⁴best response to treatment including partial and complete remission; ⁵reported as time-to-progression. | | | | | | |

As expected from the study design, there was no significant correlation between gene expression and response to therapy (RRM1, r = 0.290, p=0.053; ERCC1, r = -0.101, p=0.508), or between gene expression and survival (RRM1 and OS, p=0.38; RRM1 and PFS, p=0.38; ERCC1 and OS, p=0.10; ERCC1 and PFS, p=0.32). There also does not appear to be a difference in OS (p=0.78) or PFS (p=0.70) for patients by the selected therapy (FIG. 2B).

**Example 4. RRM1 affects cell proliferation, apoptosis, and therapeutic efficacy**. Studies were performed to investigate the effect of RRM1 expression on proliferation, apoptosis, and therapeutic efficacy as phenotypic determinants of malignancy.

Time-lapse videography (TLV) was performed with the video camera focused on an area that contained approximately 10-15 cells. Continual recording was performed for 10 days. Using this approach, it was possible to follow the progeny of each individual cell. Many H23-R1 cells underwent apoptosis, while H23-Ct cells grew exponentially. Apoptosis was confirmed by morphological and biochemical assays. The mean inter-division time for H23-R1 was 41.9 h (standard deviation 19.0 h, 95% confidence interval 32.3 to 51.5 h), and it was 25.7 h for H23-Ct (standard deviation 3.9 h, 95% confidence interval 24.3 to 27.1 h).

To monitor cell proliferation, cell cycle distribution analyses was performed with the RRM1-transfected (H23-R1) and control cell lines (H23-Ct). The results indicate that the proportion of cells in G2-phase was significantly higher in the H23-R1 line (27%) as compared to the H23-Ct (6%) line. Clonal sublines of H23-R1 were generated by serial dilution, and a strong association between the percentage of cells in G2 and the relative expression of RRM1 as determined by quantitative RT-PCR was observed. That cells were in G2 and not in M phase was confirmed by counting metaphases manually and by FACS analysis after mithramycin staining. There was no phenotypic difference between the lines. This suggests that RRM1 overexpression predominantly delays progression through G2-phase.

The proportion of cells undergoing apoptosis in the RRM1-transfected (H23-R1) and control cell lines (H23-Ct) was determined. The proportion of apoptotic cells was determine to be significantly higher in the H23-R1 line (11%) compared to H23-Ct line (2%). In a series of Western analyses on proteins implicated in pathways leading to apoptosis, H23-R1 had a substantial increase for the proteins PARP p89 (2.2-fold; cleaved product of PARP p116; PARP=poly-ADP-ribose-polymerase), AIF (2.7-fold; AIF=apoptosis-inducing factor), and cytochrome C (1.7-fold) compared to H23-Ct. This suggests that the increased apoptosis is largely mediated through the endogenous mitochondrial pathway. There were no significant differences between the parental cell line NCI-H23 and the control cell line H23-Ct in the level of expression of these proteins.

A modified MTT assay (MTS) was used to assess the impact of RRM1 expression on therapeutic efficacy for the most commonly used agents in lung cancer therapy. We used H23-R1, a cell line with 3.5-fold overexpression of RRM1, and H23siR1 a cell line with reduced RRM1 expression (0.2-fold), was generated using siRNA technology. The expression levels were determined by real-time RT-PCR and Western blotting. RRM2 and β-actin expression were unaffected.

H23siR1 was generated by stable transfection of NCI-H23 with the pSUPER-siRRM1 construct. To generate this construct, the pSUPER-GFP/neo vector was digested with BgIII and HindII and the annealed oligonucleotides (5'-GATCC CCgac gctag agcgg tctta tTTCAAGAGA ataag accgc tctag cgtcT TTTTG GAAA-3'(SEQ ID NO:1) and 5'-AGCTT TTCCAAAAAg acgct agagc ggtct tatTC TCTTG AAata agacc gctct agcgt cGGG-3' (SEQ ID NO:2)) were ligated into the vector. RRM1 target sequences are indicated in capital letters. The H23siCt cell line was generated with the targeting sequence GCTAATAGCGCGGAGTCTT (SEQ ID NO:3), which has no similarity to any known gene.

The MTS assay was performed by seeding 4,000 cells/well in 96 well flat-bottom plates. H23-Ct, H23-R1, H23 siR1 H23siCt cells were continuously exposed to drug for 72 or 96 h. The assay was performed with 3-(4,5-dimethylthiazol-2-yl)-5-(3-carboxymethoxyphenyl)-2-(4-sulfophenyl)-2H-tetrazolium reagent. After 3 h of incubation at 37°C, the absorbance was measured at 490 nm. All experiments were repeated three times. The IC₅₀, i.e., the drug concentration that inhibits cell growth by 50%, was calculated as: [(mean absorbance in triplicate wells containing the drug - mean absorbance in triplicate blank wells) / (mean absorbance in triplicate drug-free wells - mean absorbance in triplicate blank wells)] x 100. The results are shown in Table 5.

**Table 5: IC₅₀ values of NCI-H23 and constructs with increased and decreased RRM1 expression. The values given are means +/- SD from three separate experiments each done in triplicate.**

| | Gemcitabine IC₅₀ [nM] | Cisplatin IC₅₀ [nM] | Docetaxel IC₅₀ [nM] | Vinorelbine IC₅₀ [µM] |
|---|---|---|---|---|
| NCI-H23 | 500 +/- 9.0 | 1,200 +/- 140 | 1,000 +/- 0.0 | 8.0 |
| H23-R1 | 6,000 +/- 107 | 1,500 +/- 85.0 | 9.0 +/- 7.0 | 0.4 |
| H23-Ct | 750 +/- 12.0 | 1,250 +/- 50.0 | 700 +/- 141 | 20.0 |
| H23siR1 | 100 +/- 29.0 | 950 +/- 7.0 | 6.0 +/- 3.0 | 0.01 |
| H23siCt | 1,015 +/- 140 | 1,000 +/- 210.0 | 2,500 +/- 134 | 20.0 |

Increased RRM1 expression resulted in resistance to gemcitabine. The gemcitabine IC₅₀ of H23-R1 was 8-fold higher than the IC₅₀ of H23-Ct (Table 4). The response of the parental cell line (NCI-H23) to gemcitabine was similar to the response of H23-Ct. Reduced RRM1 expression increased sensitivity to gemcitabine.

There was a similar, but small, relationship between RRM1 expression and cytotoxicity response to cisplatin. Although, the IC₅₀ of H23-R1 was slightly higher and the IC₅₀ of H23siR1 was slightly lower than the corresponding control values.

A complex cytotoxicity response as a function of RRM1 expression was found when docetaxel and vinorelbine were used. Both H23-R1 and H23siR1 were more sensitive to docetaxel and vinorelbine than the control cell lines.

Induction of apoptosis as a result of drug exposure was determined with 3x10⁵ cells/well in six-well plates after 2, 4 and 6 hours of treatment. Cells were then labeled with annexin V, and labeling was determined by flow cytometry (FACScalibur, Becton Dickinson). After 4 h and 6 h of gemcitabine treatment 2.55% and 5.05% of the H23siCt cells were apoptotic while 5.09% and 15.7% of H23siR1 cells were apoptotic (FIG. 4). Likewise, the proportion of apoptotic cells after exposure to docetaxel was higher in H23siR1 than in control cells. Docetaxel also induced more apoptosis in H23-R1 than in control cells. The increased apoptosis caused by both drugs in cell lines with reduced RRM1 expression was significant (p<0.05) as compared to control cells.

**Table 6. Summary of observations**

| | RRM1 high expression | RRM1 medium expression | RRM1 low expression | ERCC1 high expression | ERCC1 low expression |
|---|---|---|---|---|---|
| Gemcitavine (antimetabolite) | resistant | | *sensitive* | | |
| Pemetrexed (antimetabolite) | resistant | | *sensitive* | | |
| Docetaxel (antitubulin) | *Sensitive* | Resistant | *sensitize* | | |
| Vinorelbine (antitubulin) | *Sensitive* | Resistant | *sensitive* | | |
| Cisplatin (DNA cross-linking/alkylating agent) | Minimal resistance | | Minimal sensitivity | resistant | *sensitive* |
| Carboplatin (DNA cross-linking/alkylating agent) | Minimal resistance | | Minimal sensitivity | resistant | *sensitive* |

A number of embodiments of the invention have been described. Nevertheless, it will be understood that various modifications may be made without departing from the spirit and scope of the invention. Accordingly, other embodiments are within the scope of the following claims.

In particular, further aspects of the invention are set forth in the following clauses:
1. An in vitro method for assessing a subject for an appropriate chemotherapy, comprising:
   (i) providing a tumor sample from the subject;
   (ii) determining the RRM1 expression level in the tumor sample; and
   (iii) determining an appropriate chemotherapy based on the RRM1 expression level, wherein if the RRM1 expression level is less than or equal to the median RRM1 expression level of a reference cohort, then a chemotherapy comprising an antimetabolite is determined to be appropriate, and wherein if the RRM1 expression level is greater than the median RRM1 expression level of the reference cohort, then a chemotherapy lacking an antimetabolite is determined to be appropriate.
2. The method of clause 1, further comprising determining the ERCC1 expression level in the tumor sample.
3. The method of clause 2, further comprising assessing the subject for an appropriate chemotherapy based on the ERCC1 expression level, wherein if the ERCC1 expression level is less than or equal to the median ERCC1 expression level of the reference cohort, then a chemotherapy comprising a platinum-containing agent is determined to be appropriate, and if the ERCC1 expression level is greater than the median ERCC1 expression level of the reference cohort, then a chemotherapy lacking a platinum-containing agent is determined to be appropriate.
4. The method of clause 3, wherein the platinum-containing agent is cisplatin, carboplatin, or oxaliplatin.
5. The method of any one of clauses 1-4, wherein determining the RRM1 expression level comprises performing an RT-PCR, Northern blot, Western blot, or immunohistochemistry assay.
6. The method of any one of clauses 2-5, wherein determining the ERCC1 expression level comprises performing an RT-PCR, Northern blot, Western blot or immunohistochemistry assay.
7. The method of any one of clauses 1-6, further comprising selecting the appropriate chemotherapy for the subject.
8. The method of clause 7, comprising selecting an antimetabolite if the RRM1 expression level is determined to be less than or equal to the median RRM1 expression level of the reference cohort.
9. The method of clause 8, wherein the antimetabolite is gemcitabine or pemetrexed.
10. The method of any one of clauses 7-9, further comprising selecting a second chemotherapeutic agent for the subject.
11. The method of clause 10, wherein the second chemotherapeutic agent is an antitubulin or platinum-containing agent.
12. The method of clause 11, wherein the antitubulin agent is a taxane or a vinca alkaloid.
13. The method of clause 12, wherein the taxane is paclitaxel or docetaxel.
14. The method of clause 12, wherein the vinca alkaloid is vinorelbine, vincristine, vinblastine, vinflunine, or vindesine.
15. The method of clause 11, wherein the platinum-containing agent is carboplatin, cisplatin, or oxaliplatin.
16. The method of any one of clauses 3-15, further comprising selecting for the subject a platinum-containing agent if the ERCC1 expression level is determined to be less than or equal to the median ERCC1 expression level of the reference cohort.
17. The method of clause 16, wherein the platinum-containing agent is cisplatin, carboplatin, or oxaliplatin.
18. The method of clause 16, further comprising selecting an antimetabolite if the RRM1 expression level is determined to be less than or equal to the median RRM1 expression level of the reference cohort.
19. The method of clause 18, wherein the antimetabolite is gemcitabine or pemetrexed.
20. The method of any one of clauses 1-19, wherein the subject has an epithelial malignancy.
21. The method of any one of clauses 1-19, wherein the subject has a lung cancer, breast cancer, colorectal cancer, head and neck cancer, or ovarian cancer.
22. The method of any one of clauses 1-19, wherein the subject has non-small-cell lung cancer.
23. The method of any one of clauses 1-22, further comprising selecting radiation therapy for the subject.
24. An in vitro method for assessing a subject for an appropriate chemotherapy, comprising:
   (i) providing a tumor sample from the subject;
   (ii) determining the RRM1 expression level in the tumor sample; and
   (iii) determining an appropriate chemotherapy based on the RRM1 expression level, wherein if the RRM1 expression level is less than or equal to the RRM1 expression level of the lowest quartile of a reference cohort, or greater than or equal to the RRM1 expression level of the highest quartile of a reference cohort, then a chemotherapy comprising an antitubulin is determined to be appropriate, and wherein if the RRM1 expression level is not determined to be in the lowest or highest quartile of the reference cohort, then a chemotherapy lacking an antitubulin is determined to be appropriate.
25. The method of clause 24, further comprising determining the ERCC1 expression level in the tumor sample.
26. The method of clause 25, further comprising assessing the subject for an appropriate chemotherapy based on the ERCC1 expression level, wherein if the ERCC1 expression level is less than or equal to the median ERCC1 expression level of the reference cohort, then a chemotherapy comprising a platinum-containing agent is appropriate, and if the ERCC1 expression level is greater than the median ERCC1 expression level of the reference cohort, then a chemotherapy lacking a platinum-containing agent is appropriate.
27. The method of clause 24, further comprising selecting the appropriate chemotherapy for the subject.
28. The method of clause 27, further comprising selecting the appropriate chemotherapy for the subject.
29. The method of clause 24, further comprising selecting an antitubulin if the RRM1 expression level is determined to be less than or equal to the lowest quartile RRM1 expression level of the reference cohort, or if the RRM1 expression level is determined to be greater than or equal to the highest quartile RRM1 expression level of the reference cohort.
30. The method of clause 29, wherein the antitubulin is a taxane or a vinca alkaloid.
31. The method of clause 30, wherein the taxane is paclitaxel or docetaxel.
32. The method of clause 30, wherein the vinca alkaloid is vinorelbine, vincristine, vinblastine, vinflunine, or vindesine.
33. The method of clause 26, further comprising selecting a platinum-containing agent if the ERCC1 expression level is determined to be less than or equal to the median ERCC1 expression level of the reference cohort.
34. The method of clause 33, wherein the platinum-containing agent is cisplatin, carboplatin, or oxaliplatin.
35. The method of any one of clauses 24-34, wherein the subject has an epithelial malignancy.
36. The method of any one of clauses 24-35, further comprising selecting radiation therapy for the subject.
37. The method of any one of clauses 24-36, further comprising selecting surgery for the subject.
38. A method for assessing the efficacy of a composition comprising a chemotherapeutic agent, the method comprising:
   (i) providing a first cell line and a second cell line, wherein the first cell line expresses higher levels of RRM1 than the second cell line;
   (ii) contacting the first and second cell lines with the composition for a time sufficient to affect cell viability; and
   (iii) assaying the first and second cell lines for an effect on cell viability, wherein a decrease in cell viability in the second cell line as compared to the first cell line indicates that the composition is likely to be efficacious against a tumor expressing lower levels of RRM1.
39. The method of clause 38, wherein the composition comprises one or more of an antimetabolite, an antitubulin, or a platinum-containing agent.
40. The method of clause 39, wherein the antimetabolite is gemcitabine or pemetrexed.
41. The method of clause 39, wherein the antitubulin is a taxane or a vinca alkaloid.
42. The method of clause 41, wherein the taxane is paclitaxel or docetaxel.
43. The method of clause 41, wherein the vinca alkaloid is vinorelbine, vincristine, vinblastine, vinflunine, or vindesine.
44. The method of clause 39, wherein the platinum-containing agent is carboplatin, cisplatin, or oxaliplatin.
45. A method for assessing the efficacy of a composition comprising a chemotherapeutic agent, the method comprising:
   (i) providing a first cell line and a second cell line, wherein the first cell line expresses higher levels of RRM1 than the second cell line;
   (ii) contacting the first and second cell lines with the composition for a time sufficient to affect cell viability; and
   (iii) assaying the first and second cell lines for an effect on cell viability, wherein a decrease in cell viability in the first cell line as compared to the second cell line indicates that the composition is likely to be efficacious against a tumor expressing higher levels of RRM1.
46. A method for assessing the efficacy of a composition comprising a chemotherapeutic agent, the method comprising:
   (i) providing a first cell line and a second cell line, wherein the first cell line expresses higher levels of ERCC1 than the second cell line;
   (ii) contacting the first and second cell lines with the composition for a time sufficient to affect cell viability; and
   (iii) assaying the first and second cell lines for an effect on cell viability, wherein a decrease in cell viability in the second cell line as compared to the first cell line indicates that the composition is likely to be efficacious against a tumor expressing lower levels of ERCC1.
47. The method of clause 46, wherein the composition comprises one or more of an antimetabolite, an antitubulin, or a platinum-containing agent.
48. The method of clause 47, wherein the antimetabolite is gemcitabine or pemetrexed.
49. The method of clause 47, wherein the antitubulin is a taxane or a vinca alkaloid.
50. The method of clause 47, wherein the platinum-containing agent is carboplatin, cisplatin, or oxaliplatin.
51. The method of clause 49, wherein the taxane is paclitaxel or docetaxel.
52. The method of clause 49, wherein the vinca alkaloid is vinorelbine, vincristine, vinblastine, vinflunine, or vindesine.
53. A method for assessing the efficacy of a composition comprising a chemotherapeutic agent, the method comprising:
   (i) providing a first cell line and a second cell line, wherein the first cell line expresses higher levels of ERCC1 than the second cell line;
   (ii) contacting the first and second cell lines with the composition for a time sufficient to affect cell viability; and
   (iii) assaying the first and second cell lines for an effect on cell viability, wherein a decrease in cell viability in the first cell line as compared to the second cell line indicates that the composition is likely to be efficacious against a tumor expressing higher levels of ERCC1.
54. A kit comprising:
   (i) a reagent for assaying RRM1 expression in a tissue sample from a patient, and
   (ii) an instruction sheet.
55. The kit of clause 54, wherein the reagent for assaying RRM1 expression comprises a premeasured portion of a reagent selected from the group selected from oligo-dT primers, forward primers that hybridize to an RRM1 cDNA, reverse primers that hybridize to an RRM1 cDNA, reverse transcriptases, DNA polymerases, buffers, and nucleotides.
56. The kit of clause 54, wherein the reagent for assaying RRM1 expression comprises a premeasured portion of an anti-RRM1 antibody and buffers for performing a Western blot or immunohistochemistry assay.
57. The kit of clause 54, further comprising a reagent for processing a tissue sample from a patient.
58. The kit of clause 54, further comprising a reagent for assaying ERCC1 expression in the biopsy tissue.
59. A kit comprising:
   (i) a reagent for assaying ERCC1 expression in a tissue sample from a patient, and
   (ii) an instruction sheet.
60. The kit of clause 59, wherein the reagent for assaying ERCC1 expression comprises a premeasured portion of a reagent selected from the group selected from oligo-dT primers, forward primers that hybridize to an ERCC1 cDNA, reverse primers that hybridize to an ERCC1 cDNA, reverse transcriptases, DNA polymerases, buffers, and nucleotides.
61. The kit of clause 59, wherein the reagent for assaying ERCC1 expression comprises a premeasured portion of an anti-ERCC1 antibody and buffers for performing a Western blot or immunohistochemistry assay.
62. The kit of clause 59, further comprising a reagent for processing a tissue sample from a patient.
63. A kit comprising:
   (i) a reagent for assaying RRM1 expression in a tissue sample from a patient,
   (ii) a reagent for assaying ERCC1 expression in a tissue sample from a patient, and
   (ii) an instruction sheet.

## Claims

1. An in vitro method for assessing a subject for an appropriate chemotherapy, comprising:
(i) providing a tumor sample from the subject;
(ii) determining the RRM1 expression level in the tumor sample; and
(iii) determining an appropriate chemotherapy based on the RRM1 expression level, wherein if the RRM1 expression level is less than or equal to the median RRM1 expression level of a reference cohort, then a chemotherapy comprising an antimetabolite is determined to be appropriate, and wherein if the RRM1 expression level is greater than the median RRM1 expression level of the reference cohort, then a chemotherapy lacking an antimetabolite is determined to be appropriate.

2. The method of claim 1, further comprising determining the ERCC1 expression level in the tumor sample.

3. The method of claim 2, further comprising assessing the subject for an appropriate chemotherapy based on the ERCC1 expression level, wherein if the ERCC1 expression level is less than or equal to the median ERCC1 expression level of the reference cohort, then a chemotherapy comprising a platinum-containing agent is determined to be appropriate, and if the ERCC1 expression level is greater than the median ERCC1 expression level of the reference cohort, then a chemotherapy lacking a platinum-containing agent is determined to be appropriate.

4. The method of claim 3, wherein the platinum-containing agent is cisplatin, carboplatin, or oxaliplatin.

5. The method of any one of claims 1-4, wherein determining the RRM1 expression level comprises performing an RT-PCR, Northern blot, Western blot, or immunohistochemistry assay.

6. An in vitro method for assessing a subject for an appropriate chemotherapy, comprising:
(i) providing a tumor sample from the subject;
(ii) determining the RRM1 expression level in the tumor sample; and
(iii) determining an appropriate chemotherapy based on the RRM1 expression level, wherein if the RRM1 expression level is less than or equal to the RRM1 expression level of the lowest quartile of a reference cohort, or greater than or equal to the RRM1 expression level of the highest quartile of a reference cohort, then a chemotherapy comprising an antitubulin is determined to be appropriate, and wherein if the RRM1 expression level is not determined to be in the lowest or highest quartile of the reference cohort, then a chemotherapy lacking an antitubulin is determined to be appropriate.

7. The method of claim 6, further comprising determining the ERCC1 expression level in the tumor sample.

8. The method of claim 7, further comprising assessing the subject for an appropriate chemotherapy based on the ERCC1 expression level, wherein if the ERCC1 expression level is less than or equal to the median ERCC1 expression level of the reference cohort, then a chemotherapy comprising a platinum-containng agent is appropriate, and if the ERCC1 expression level is greater than the median ERCC1 expression level of the reference cohort, then a chemotherapy lacking a platinum-containing agent is appropriate.

9. The method of claim 6, further comprising selecting an antitubulin if the RRM1 expression level is determined to be less than or equal to the lowest quartile RRM1 expression level of the reference cohort, or if the RRM1 expression level is determined to be greater than or equal to the highest quartile RRM1 expression level of the reference cohort.

10. The method of claim 9, wherein the antitubulin is a taxane, e.g., paclitaxel or docetaxel, or a vinca alkaloid, e.g., vinorelbine, vincristine, vinblastine, vinflunine, or vindesine.

11. The method of claim 8, wherein the platinum-containing agent is cisplatin, carboplatin, or oxaliplatin.

12. A method for assessing the efficacy of a composition comprising a chemotherapeutic agent, the method comprising:
(i) providing a first cell line and a second cell line, wherein the first cell line expresses higher levels of RRM1 than the second cell line;
(ii) contacting the first and second cell lines with the composition for a time sufficient to affect cell viability; and
(iii) assaying the first and second cell lines for an effect on cell viability, wherein a decrease in cell viability in the second cell line as compared to the first cell line indicates that the composition is likely to be efficacious against a tumor expressing lower levels of RRM1.

13. A method for assessing the efficacy of a composition comprising a chemotherapeutic agent, the method comprising:
(i) providing a first cell line and a second cell line, wherein the first cell line expresses higher levels of RRM1 than the second cell line;
(ii) contacting the first and second cell lines with the composition for a time sufficient to affect cell viability; and
(iii) assaying the first and second cell lines for an effect on cell viability, wherein a decrease in cell viability in the first cell line as compared to the second cell line indicates that the composition is likely to be efficacious against a tumor expressing higher levels of RRM1.

14. A method for assessing the efficacy of a composition comprising a chemotherapeutic agent, the method comprising:
(i) providing a first cell line and a second cell line, wherein the first cell line expresses higher levels of ERCC1 than the second cell line;
(ii) contacting the first and second cell lines with the composition for a time sufficient to affect cell viability; and
(iii) assaying the first and second cell lines for an effect on cell viability, wherein a decrease in cell viability in the second cell line as compared to the first cell line indicates that the composition is likely to be efficacious against a tumor expressing lower levels of ERCC1.

15. A method for assessing the efficacy of a composition comprising a chemotherapeutic agent, the method comprising:
(i) providing a first cell line and a second cell line, wherein the first cell line expresses higher levels of ERCC1 than the second cell line;
(ii) contacting the first and second cell lines with the composition for a time sufficient to affect cell viability; and
(iii) assaying the first and second cell lines for an effect on cell viability, wherein a decrease in cell viability in the first cell line as compared to the second cell line indicates that the composition is likely to be efficacious against a tumor expressing higher levels of ERCC1.
